**Europäisches Patentamt**

**European Patent Office**

**Office européen des brevets**

(19)

(11) Veröffentlichungsnummer: **0 169 975**
**B1**

(12) **EUROPÄISCHE PATENTSCHRIFT**

(45) Veröffentlichungstag der Patentschrift:
10.08.88

(21) Anmeldenummer: 85104236.6

(22) Anmeldetag: 06.04.85

(51) Int. Cl.⁴: **A 61 F 2/34**

(54) **Randlose Endoprothese für eine Hüftgelenkspfanne.**

(30) Priorität: 03.07.84 CH 3183/84

(43) Veröffentlichungstag der Anmeldung:
05.02.86 Patentblatt 86/6

(45) Bekanntmachung des Hinweises auf die Patenterteilung:
10.08.88 Patentblatt 88/32

(84) Benannte Vertragsstaaten:
AT DE FR GB IT

(56) Entgegenhaltungen:
FR-A-2 225 924
US-A-3 685 058
US-A-3 840 904

(73) Patentinhaber: **GEBRÜDER SULZER AKTIENGESELLSCHAFT**, Zürcherstrasse 9, CH-8401 Winterthur (CH)

(72) Erfinder: **Griss, Peter, Prof. Dr.- Med., Klinikum Mannheim, D-6800 Mannheim (DE)**

(74) Vertreter: **Dipl.- Ing. H. Marsch Dipl.- Ing. K. Sparing Dipl.- Phys.Dr. W.H. Röhl Patentanwälte, Rethelstrasse 123, D-4000 Düsseldorf (DE)**

EP 0 169 975 B1

## Beschreibung

Die Erfindung betrifft eine randlose Endoprothese für eine Hüftgelenkspfanne mit einer mindestens annähernd halbkugelförmigen Außenoberfläche, aus der mindestens ein Verankerungszapfen hervorspringt, dessen Achse mit der Rotationssymmetrieachse des Pfannenkörpers einen spitzen Winkel bildet (z. B. US-A-3 685 058).

Randlose Endoprothesen der genannten Art sind bereits vorgeschlagen worden; sie werden in neuerer Zeit vor allem für eine zementfreie Verankerung benutzt, wobei sie entweder in die Beckenknochen eingeschraubt oder eingeschlagen werden. Das Einschrauben der Prothesen ist für den Operateur wegen des zu schneidenden Gewindes mühsam und zeitraubend. Bei einem zementfreien Einpressen oder Einschlagen der genannten Pfannenprothesen, das in Richtung der Zapfenachse bzw. der die Zapfenachsen enthaltenden Ebene erfolgt, haben sich Schwierigkeiten sowohl bei der Primär-Stabilität als auch bei der Dauerverankerung ergeben. Diese Schwierigkeiten resultieren aus den infolge von Reibungskräften auftretenden Biege- und Kippmomenten, durch die vor allem die Wurzel des oder der Zapfen belastet werden.

Aufgabe der Erfindung ist es, zum einen die Übertragung der Kipp- und Biegemomente auf den Knochen zu verbessern und so die Lockerungstendenzen bei derartigen Pfannenprothesen zu verringern und zum anderen die Primär-Verankerung in der Anfangszeit nach der Implantation zu verbessern. Diese Aufgabe wird mit der vorliegenden Erfindung dadurch gelöst, daß an den Zapfen im Wurzelbereich Lappen angesetzt sind, deren Dachkanten als Schneiden ausgebildet sind.

Das Eindringen der messerartigen Schneiden in den Knochen ergibt eine gleichmässige Verdichtung des dadurch verdrängten Knochengewebes, wodurch in erster Linie die Primär-Stabilität verbessert wird. Die Anordnung der Lappen im Wurzelbereich des Zapfens entlastet diesen entscheidend, da dadurch eine erhebliche Vergrösserung der "Übertragungs"-Fläche erreicht wird.

Das Eindringen der Lappen in den Knochen kann während der Implantation erleichtert werden, wenn die Dachkante von den Lappen nach außen gegen die Außenoberfläche abfällt.

Die neue Endoprothese kann aus allen für Pfannenprothesen gebräuchlichen Implantat-Werkstoffen gefertigt sein. Vorzugsweise besteht sie jedoch aus Metall, z. B. Titan oder einer Titanlegierung.

Um die Sekundär-Verankerung zu verbessern, ist es selbstverständlich möglich, die Außenfläche des Pfannenkörpers und/oder den Zapfen in bekannter Weise mit Verankerungsstrukturen, wie Rillen, Noppen, Rippen usw., zu versehen.

Im folgenden wird die Erfindung anhand eines Ausführungsbeispiels im Zusammenhang mit der Zeichnung näher erläutert.

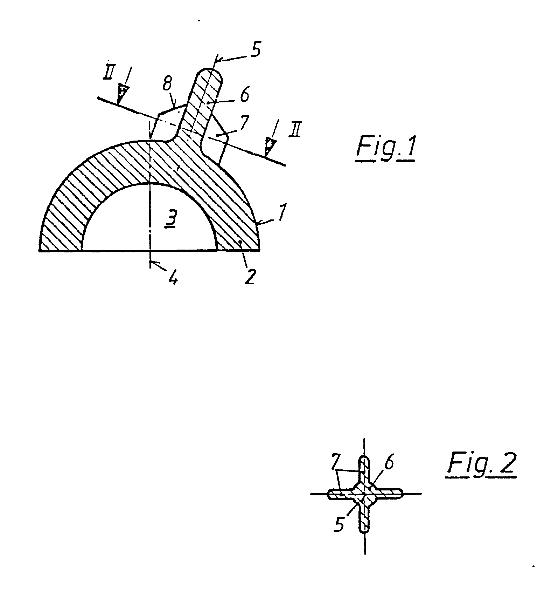

Fig. 1 ist ein Schnitt durch die neue Prothese, der in der durch die Zapfen- und die Rotationssymmetrieachse des Pfannenkörpers festgelegten Meridianebene des halbkugelförmigen Pfannenkörpers geführt ist;

Fig. 2 ist der Schnitt II-II von Fig. 1.

Sowohl die Außenfläche (1) eines der Pfannenkörper 2 als auch die eigentliche Pfannenschale 3, die den nicht dargestellten Gelenkkopf einer Femurkopfprothese aufnimmt, sind bezüglich der Achse 4 rotationssymmetrisch gestaltet.

Mit dieser Achse 4 bildet eine Achse 5 eines Verankerungszapfens 6, der in Richtung der Hauptbelastung der Endoprothese - die auch die Einschlagrichtung ist - verläuft, einen spitzen Winkel zwischen 20° und 30°.

Nach der vorliegenden Erfindung sind in dem gezeigten Beispiel - vier Lappen oder Flügel 7 im Wurzelbereich des Zapfens 6 vorgesehen, die paarweise aufeinander senkrecht stehen. Die Dachkante 8 der Lappen 7, die als messerartige Schneide ausgebildet ist, fällt vom Zapfenmantel aus nach aussen leicht ab, wodurch - wie erwähnt - das Eindringen in den Knochen erleichtert wird.

Die Erfindung ist nicht auf das beschriebene Ausführungsbeispiel beschränkt; insbesondere können statt eines Zapfens 6 zwei mit ihren Achsen 5 parallel zueinander in einer Ebene verlaufende Zapfen vorgesehen sein. Weiterhin können die Oberfläche des Pfannenkörpers 2 und/oder des Zapfens 6 mit einer das Ein- und Anwachsen von Gewebe verbessernden Struktur versehen sein, die in bekannter Weise aus Rillen, Rippen, Noppenvertiefungen, Lappen usw. besteht.

## Patentansprüche

1. Randlose Endoprothese für eine Hüftgelenkspfanne (2) mit einer mindestens annähernd halbkugelförmigen Außenoberfläche, aus der mindestens ein Verankerungszapfen (6) hervorspringt, dessen Achse (5) mit der Rotationssymmetrieachse des Pfannenkörpers einen spitzen Winkel bildet, dadurch gekennzeichnet, daß an den Zapfen (6) im Wurzelbereich Lappen (7) angesetzt sind, deren Dachkanten (8) als Schneiden ausgebildet sind.

2. Endoprothese nach Anspruch 1, dadurch gekennzeichnet, daß die Dachkante (8) von den Lappen (7) nach aussen gegen die Aussenoberfläche (1) abfällt.

## Claims

1. An edgeless endoprosthesis for an acetabulum replacement (2) having an at least substantially hemispherical outside surface from which at least one fixing nail (6) projects, the nail axis (5) forming an acute angle with the axis of rotational symmetry of the replacement (2), characterised in that fins or ridges (7) or the like project from the root zone of the nail (6) and have inclined edges (8) in the form of cutting edges.

2. An endoprosthesis according to claim 1, characterised in that the inclined edge (8) descends outwardy from the fins (7) towards the outside surface (1).

## Revendications

1. Endoprothèse dépourvue de collerette pour une cuvette (2) d'articulation coxofémorale, présentant une surface extérieure au moins approximativement hémisphérique d'où fait saillie au moins un tenon d'ancrage (6), dont l'axe (5) forme un angle aigu avec l'axe de symétrie de rotation du corps de la cuvette, caractérisée par le fait que des oreilles (7) sont implantées dans la région de la racine du tenon (6), les arêtes prismatiques (8) de ces oreilles étant réalisées sous la forme de tranchants.

2. Endoprothèse selon la revendication 1, caractérisée par le fait que l'arête prismatique (8) décline vers l'extérieur, à partir des oreilles (7), en direction de la surface extérieure (1).

*Fig.1*

*Fig.2*